# EUROPEAN PATENT APPLICATION

(11) **EP 1 806 587 A1**
(43) Date of publication of application: **11.07.2007**
(21) Application number: 06100141.8
(22) Date of filing: 07.01.2006
(51) Int. Cl.: G01N 33/68

(54) **An in-vitro method for screening accessible biological markers in pathologic tissues**

(71) Applicant: Université de Liège, 4020 Liège (BE)
(72) Inventor: Castronovo, Vincent, c/o Université de Liége, 4000 Liége (BE); Waltregny, David, c/o Université de Liége, 4000 Liége (BE); Kischel, Philippe, c/o Université de Liége, 4000 Liége (BE)
(74) Representative: polypatent

(57) **Abstract**

The present invention refers to an in-vitro method for screening specific disease biological markers accessible from the extra cellular space in pathologic tissues comprising the steps of:
- immersion of a pathologic tissue sample in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to normal tissue samples;
- judging that the labelled protein(s) having higher expression in the pathologic tissue sample compared to normal tissue samples or being expressed more frequently in respective pathologic tissue samples compared to normal tissue samples is/are accessible biological marker(s) for pathologic tissue.

## Description

The present invention refers to an in-vitro method for screening specific disease biological markers accessible from the extra cellular space in pathologic tissues.

The discovery of biological markers clinically suited for the accurate detection and selective treatment of diseases represents a major medical challenge. Targeting pathologic tissues without affecting their normal counterparts is one of the most promising approaches for improving treatment efficiency and safety (Wu, A. M. & Senter, P. D. Arming antibodies: prospects and challenges for immunoconjugates. Nat Biotechnol 23, 1137-1146 (2005); Adams, G. P. & Weiner, L. M. Monoclonal antibody therapy of cancer. Nat Biotechnol23, 1147-1157 (2005); Carter, P. Improving the efficacy of antibody-based cancer therapies. Nat Rev Cancer1, 118-129 (2001)). Indisputably, such advances would represent a breakthrough for the therapy of cancer and other diseases. Hence, the identification of valid biological markers including proteins specifically expressed in diseased tissues, has become of utmost importance (Hortobagyi, G. N. Opportunities and challenges in the development of targeted therapies. Semin Oncol 31, 21-27 (2004); Neri, D. & Bicknell, R. Tumour vascular targeting. Nat Rev Cancer5, 436-446 (2005)). Yet, even the most recent target discovery strategies based on state-of-the-art, high-throughput technologies have not addressed the limitation, in human tissues, of antigen accessibility to suitable high-affinity ligands such as human monoclonal antibodies bound to bioactive molecules (Adams, G. P. & Weiner, L. M. Monoclonal antibody therapy of cancer. Nat Biotechnol 23, 1147-1157 (2005); Neri, D. & Bicknell, R. Tumour vascular targeting. Nat Rev Cancer 5, 436-446 (2005).

The identification of biological markers unique to specific pathologic processes would be most valuable for the accurate detection (*e.g.* by imaging studies) and selective therapy of diseases including cancer. For instance, patients suffering from cancer would certainly benefit from such developments. Indeed, chemotherapeutic agents, usually designed to take advantage of tumor cell characteristics such as high proliferation rates, unfortunately also target normal cycling cells including hematopoietic progenitors. The targeted delivery of these drugs and other bioactive molecules (*e.g.* radioisotopes, cytokines) to the tumor microenvironment (*e.g.* proteins specifically expressed in the stromal or vascular compartment of the tumor) by means of binding molecules such as recombinant human antibodies, would represent a considerable therapeutic improvement. This selective strategy would increase the amount of drugs reaching the tumor with little or no toxicity to the host's healthy tissues. The recent development of high-throughput proteomic technologies such as mass spectrometry have facilitated the rapid and accurate identification of small, but complex biological sample mixtures, overcoming many limitations of two-dimensional gel electrophoresis for proteome analysis (Peng, J. & Gygi, S. P. Proteomics: the move to mixtures. J Mass Spectrom 36, 1083-91 (2001)) and making target identification easier than ever. The recent development of this high-throughput proteomic technology holds great promise for speeding up the discovery of novel targets, notably in cancer research. For example, gel-free shotgun tandem mass spectrometry has been recently used to compare global protein expression profiles in human mammary epithelial normal and cancer cell lines (Sandhu, C. et al., Global protein shotgun expression profiling of proliferating mcf-7 breast cancer cells. J Proteome Res 4, 674-89 (2005)). Unfortunately, a significant pitfall associated with such an approach is that it provides no clue as to whether proteins of interest are accessible to suitable high-affinity ligands, such as systemically delivered monoclonal antibodies, in human tissues. Indeed, specific, yet poorly accessible proteins expressed in pathologic tissues are expected to be of little value for the development of antibody-based anti-cancer therapies. Strategies that would unveil disease biological markers not only specifically expressed in pathologic tissues but also accessible from the extracellular fluid would help overcome this limitation.

Further, methods are known allowing protein labelling either by *in vivo* terminal perfusion or by *ex vivo* perfusion of human pathologic organs. However, the perfusion technique is restricted to experimental animals (e.g. rodents) or surgically resected organs vascularized by a catheterizable artery (e.g. kidney), which excludes many types of pathologic tissues from investigation, namely those which can not be perfused.

The object of the present invention therefore was to provide a new, simple, quick and efficient method to identify, in human tissues originating from biopsies and non-perfusable organs (e.g. cancer lesions present in mastectomy or radical prostatectomy specimens), specific disease biological markers accessible from the extracellular space.

The object is solved by an in vitro method for screening specific disease biological markers accessible from the extra cellular space in pathologic tissues comprising the steps of:
- immersion of a pathologic tissue sample in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to normal tissue;
- judging that the labelled protein(s) having higher expression in the pathologic tissue sample compared to normal tissue or being expressed more frequently in respective pathologic tissue samples compared to normal tissue is/are accessible biological marker(s) for pathologic tissue.

The present invention therefore provides a new chemical proteomic method for the reliable identification of target proteins from diseased tissues, which are accessible from the extra cellular fluid and thus from the bloodstream. By applying this procedure, for example, to small, surgically obtained samples of normal and cancerous human breast tissues, a series of accessible proteins selectively expressed in breast cancer are identified. Some of these breast cancer-associated antigens correspond to extracellular proteins including extra cellular matrix and secreted proteins, and appear to be interesting targets for antibody-based anti-cancer treatments. This powerful technology can theoretically be applied to virtually any tissue and pathologic condition and may become the basis of custom-made target therapies.

An important feature of the present invention is that the tissue samples are immersed in a solution comprising a reactive compound which is marking and labelling proteins which are accessible from the extra cellular space, by simple diffusion. This means that once the tissue sample is immersed into the solution the reactive compound comprised therein will diffuse through the extracellular space of the tissue sample and thereby brought into contact with accessible proteins and react with them. The labelled proteins can be easily purified and analysed and identified by proteomic methods. Identification of the labelled proteins is, for example, achieved by liquid chromatography and subsequent mass spectrometry. One major advantage is that the present method does not depend on the possibility that the tissue samples used can be perfused or not. Therefore, according to the method of the present invention any tissue can be investigated and explored in order to seek for accessible biological markers.

As used herein the term "biological marker" represents proteins or polypeptides (which may be modified for example by glycosylation), which are expressed in a given pathologic tissue and which are essentially not expressed in normal tissues, or are expressed on a higher level in the given pathologic tissue than in normal tissues, wherein the biological marker indicates a pathologic condition compared to the normal physiologic condition of normal tissues.

As used herein the term "normal tissue" represents either normal tissue corresponding to the pathologic tissue from the same individual or normal tissue corresponding to the pathologic tissue from other individuals or normal tissue that is not related (in extenso either from a different location in the body, or with a different histologic type) to the pathologic tissue either from the same individual or form other individuals.

In a preferred embodiment the term "normal tissue" refers to the normal tissue corresponding the pathologic tissue from the same individual.

According to the present invention the step of determination of the "differential expression pattern" of the labelled proteins in pathologic tissue samples compared to normal tissue samples means that it is examined, for example, if a given labelled protein is at all expressed in a tissue or not, either pathologic or normal. This analysis may also comprise the assessment of the relation of expression level in pathologic tissue versus normal tissue. Further, this analysis may also comprise the assessment of in how many samples (eventually from different individuals) of a given type of pathologic tissue expression of a given protein is found compared to in how many samples (eventually from different indivuduals) of the given type of the corresponding or unrelated normal tissue expression of a given protein is found.

According to the present invention there is a step of judging that the labelled protein(s) having higher expression in the pathologic tissue samples compared to normal tissue samples or being expressed more frequently in respective pathologic tissue samples compared to normal tissue samples is/are accessible biological marker(s) for pathologic tissue. Most preferred, it is judged that the labelled protein(s) having expression in the pathologic tissue sample but which is not or essentially not expressed in the corresponding and/or unrelated normal tissue is/are accessible biological marker(s) for pathologic tissue

In a preferred embodiment the method comprises the steps of:
- immersion of a normal tissue sample in a solution containing a labelling reagent for labelling proteins; wherein accessible proteins are labelled by the labelling reagent;
- immersion of a pathologic tissue sample in a solution containing a labelling reagent for labelling proteins, wherein accessible proteins are labelled by the labelling reagent;
- separate purification of the labelled proteins of each of the samples;
- analysis of the labelled proteins or fragments thereof of normal tissue and pathologic tissue, respectively;
- determination of the differential expression pattern of the labelled proteins in the pathological tissue samples compared to the normal tissue samples;
- judging that the labelled protein(s) having higher expression in the pathologic tissue sample compared to the normal tissue sample or being expressed more frequently in respective pathologic tissue samples compared to the normal tissue sample is/are accessible biological marker(s) for pathologic tissue.

Further preferred the labelling reagent for labelling proteins is a reactive biotin, preferably a biotin reactive ester derivative.

In yet another preferred embodiment the purification step makes use of the label of the labelled proteins as selective marker. It is preferred that the labelled proteins are purified by affinity purification mediated by the label.

Preferably, the label is a biotin residue and purification is performed using streptavidin bound to a resin, wherein the biotin-labelled proteins are bound to the resin via streptavidin.

According to a further preferred embodiment after the purification step the labelled proteins are cleaved to peptides, preferably by proteolytic digestion.

Further preferred, the analysis step comprises mass spectrometry, preferably microsequencing by tandem mass spectrometry.

Preferably, the pathologic tissue sample is derived from tissues selected from the group consisting of tumor tissue, inflamed tissue, atheromatotic tissue or resulting from degenerative, metabolic and genetic diseases.

Further preferred, accessibility of the biological markers refers to being accessible for substances from the extra cellular space. This means that substances can diffuse *in vitro* through the extra cellular space of a given tissue to accessible biological markers. In consequence, if a specific substance for labelling is able to diffuse *in vitro* through the extra cellular space of a given pathological tissue and reaches the biological marker, which is indicating the pathologic condition, then said biological marker (which can be determined according to the present invention) likely will also be accessible *in vivo* via the extracellular space (extra cellular liquid) and therefore can be considered to be a candidate target protein (biological marker) for detecting the disease (for detecting the presence (diagnosis), and/or evaluating the spread (staging) and/or assessing the evolution (monitoring)of the disease) and for targeting drugs to the cells and tissues expressing said accessible protein (biological marker). Preferably, the substances which can diffuse *in vitro* through the extra cellular space of a given tissue to accessible biological markers are selected from the group consisting of antibodies, antibody fragments, drugs, prodrugs, ligands, biotin, and derivatives and conjugates thereof, preferably conjugates of antibodies or antibody fragments with drugs or prodrugs.

In a further preferred embodiment the biological markers are proteins or polypeptides, which are expressed in the given pathologic tissue and not expressed in the corresponding normal tissue, or are expressed on a higher level in the given pathologic tissue than in the corresponding normal tissue, wherein the biological marker indicates a pathologic condition compared to the normal physiologic condition of the corresponding normal tissue.

The object of the present invention is also solved by the use of the aforementioned method for the manufacture of a medicament for therapeutic and/or preventive treatment of a human or animal disease, wherein a high-affinity ligand directed against a biological marker for pathological tissue is comprised in the medicament, wherein said biological marker is accessible for high-affinity ligands from the extra cellular space. Preferably, the high-affinity ligand is an antibody, more preferred a monoclonal antibody or a recombinant antibody, directed to the biological marker. Preferably, a method is provided for the manufacture of a medicament for therapeutic and/or preventive treatment of a human or animal disease, wherein a high-affinity ligand directed against a biological marker for pathological tissue is comprised in the medicament, wherein said biological marker is accessible for high-affinity ligands from the extra cellular space, wherein the method is comprising the steps of:
- immersion of a pathologic tissue sample in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to normal tissue;
- judging that the labelled protein(s) having higher expression in the pathologic tissue sample compared to normal tissue or being expressed more frequently in respective pathologic tissue samples compared to normal tissue is/are accessible biological marker(s) for pathologic tissue.
- raising antibodies against said biological marker and conjugation with a suitable drug for treatment of the pathologic cells and tissues by drug targeting.

The present invention also provides the use of the aforementioned method for the development of techniques for the detection of pathologic tissues. Preferably, subsequent to the choice of the biological marker (judgement step) according to the present invention antibodies are raised against the biological marker and conjugated with a detectable label well-known in the art, which is detectable, for example by scintigraphy, PET scan, NMR or X-rays. Preferably, a method is provided for the development of techniques for the detection of pathologic tissues, wherein the method is comprising the steps of:
- immersion of a pathologic tissue sample in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to normal tissue;
- judging that the labelled protein(s) having higher expression in the pathologic tissue sample compared to normal tissue or being expressed more frequently in respective pathologic tissue samples compared to normal tissue is/are accessible biological marker(s) for pathologic tissue.
- raising antibodies against said biological marker and conjugation with a detectable label, which is detectable by scintigraphy, PET scan, NMR or X-rays.

The present invention further provides the use of the aforementioned method for the development of a medicament based on the use of specific ligands, preferably antibodies and antibody-drug conjugates, for therapeutic and/or preventive treatment of human or animal diseases. Preferably, subsequent to the choice of the biological marker (judgement step) according to the present invention antibodies are raised against the biological marker and conjugated with a suitable drug for treatment the pathologic cells and tissues by drug targeting. Preferably, a method is provided for the development of a medicament based on the use of specific ligands, preferably antibodies and antibody-drug conjugates, for therapeutic and/or preventive treatment of human or animal diseases, wherein the method is comprising the steps of:
- immersion of a pathologic tissue sample in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to normal tissue;
- judging that the labelled protein(s) having higher expression in the pathologic tissue sample compared to normal tissue or being expressed more frequently in respective pathologic tissue samples compared to normal tissue is/are accessible biological marker(s) for pathologic tissue.
- raising antibodies against said biological marker and conjugation with a suitable drug for treatment of the pathologic cells and tissues by drug targeting.

The present invention further provides the use of the aforementioned method for the screening of accessible biological markers of a pathologic tissue of an individual patient for the development of an individual treatment protocol. Preferably, subsequent to the choice of the biological marker (judgement step) according to the present invention antibodies are raised against the biological marker and conjugated with a suitable drug for treatment the pathologic cells and tissues by drug targeting. Preferably, a method is provided for the development of a medicament based on the use of specific ligands, preferably antibodies and antibody-drug conjugates, for therapeutic and/or preventive treatment of human or animal diseases, wherein the method is comprising the steps of:
- immersion of a pathologic tissue sample of an individual patient in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to normal tissue;
- judging that the labelled protein(s) having higher expression in the pathologic tissue sample compared to normal tissue or being expressed more frequently in respective pathologic tissue samples compared to normal tissue is/are accessible biological marker(s) for pathologic tissue.
- raising antibodies against said biological marker and conjugation with a suitable drug for treatment of the pathologic cells and tissues by drug targeting.

The object is also solved by a method for therapeutic and/or preventive treatment of a human or animal disease, wherein a high-affinity ligand directed against a biological marker for pathological tissue is used, wherein said biological marker is accessible for high-affinity ligands from the extra cellular space, wherein the method is comprising the aformentioned procedure of the present invention. Preferably, a method is provided for therapeutic and/or preventive treatment of a human or animal disease, wherein a high-affinity ligand directed against an biological marker for pathological tissue is used, wherein said biological marker is accessible for high-affinity ligands from the extra cellular space, wherein the method is comprising the steps of:
- immersion of a pathologic tissue sample in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to normal tissue;
- judging that the labelled protein(s) having higher expression in the pathologic tissue sample compared to normal tissue or being expressed more frequently in respective pathologic tissue samples compared to normal tissue is/are accessible biological marker(s) for pathologic tissue;
- raising antibodies against said biological marker and conjugation with a suitable drug for treatment of the pathologic cells and tissues by drug targeting.

In the present patent application a new, simple, quick and efficient method is provided to identify, in human tissue biopsies, specific disease biological markers accessible from the extra cellular space. The method of the present invention preferably makes use of covalent linking of biotin onto primary amines of accessible proteins brought into contact with a solution of reactive biotin ester derivatives. The method according to the present invention permits the *ex vivo* biotinylation of human tissues, which originate either from biopsies or from non-perfusable organs (e.g. cancer lesions present in mastectomy or prostatectomy samples), by simple immersion in the biotinylation solution. Biotinylated proteins are easily purified thanks to the extremely high and specific interaction of biotin with streptavidin, even in lysis buffers containing strong detergents, thus minimizing the non-specific binding during the affinity purification step. Proteolytic digestion of the purified biotinylated proteins preferably is performed directly on-resin, before shotgun mass spectrometry analysis.

In summary, the method of the present invention allows the identification of several accessible proteins differentially expressed in pathologic and healthy tissue samples. This method isapplicable to virtually any tissue, including tumor tissue samples as well as other pathologic tissues resulting from inflammatory, degenerative, metabolic, and even genetic diseases. The present invention sought for specific biological markers of human breast cancer, the most frequent form of cancer and the second leading cause of cancer death in American women. Among a list of candidate markers, two of them, versican and periostin, were identified in breast cancers with the chemical proteomic-based approach of the present invention, and were further validated by immunohistochemistry. The accessibility of these extra cellular matrix proteins may be particularly suited for targeting strategies using an intravenous route. It was demonstrated that the method is easy to perform and reliable. Of note, results can be obtained in less than one week. The method is versatile enough to be exploited in the future for complete mapping of primary tumors and associated metastases. It would enable the development of custom-made treatments, targeting only accessible proteins effectively expressed in the diseased tissues. It may be anticipated that once the most relevant, accessible biological markers specific for a patient's disease are identified, high affinity ligands, such as recombinant human antibodies and their fragments, can be prepared to assess the precise localization of the pathologic lesions and to selectively destroy them. As an example, the human antibody L19, a specific ligand of the EDB domain of fibronectin, a biological marker of several cancer types (Zardi, L. et al. Transformed human cells produce a new fibronectin isoform by preferential alternative splicing of a previously unobserved exon. Embo J 6, 2337-2342 (1987); Pini, A. et al. Design and use of a phage display library. Human antibodies with subnanomolar affinity against a marker of angiogenesis eluted from a two-dimensional gel. J Biol Chem 273, 21769-21776 (1998); Castellani, P. et al. Differentiation between high- and low-grade astrocytoma using a recombinant antibody to the extra domain-B of fibronectin. Am J Pathol 161, 1695-1700 (2002)), is currently tested in clinical trials, both as a imaging tool (conjugated to radioactive iodine (Berndorff, D. et al. Radioimmunotherapy of solid tumors by targeting extra domain B fibronectin: identification of the best-suited radioimmunoconjugate. Clin Cancer Res 11, 7053s-7063s (2005); Borsi, L. et al. Selective targeting of tumoral vasuclature: comparison of different formats of an antibody (L19- to the ED-B domain of fibronectin. Int J Cancer 102, 75-85 (2002)) and as a therapeutic agent (fused with human interleukin-2 (Ebbinghaus, C. et al. Engineered vascular-targeting antibody-interferon-gamma fusion protein for cancer therapy. Int J Cancer 116, 304-313 (2005); Menrad, A. & Menssen, H. D. ED-B fibronectin as a target for antibody-based cancer treatments. Expert Opin Ther Targets 9, 491-500 (2005); Carnemolia, B. et al. Enhancement of the antitumor properties of interleukin-2 by its targeted delivery to the tumor blood vessel extracellular matrix. Blood 99, 1659-1665 (2002))). The new technology of the present invention will promote the development of selective target therapies and therefore represents a major unprecedent step toward a clean and effective war against diseases and particularly cancer.

**Best Mode for carrying out the present invention**

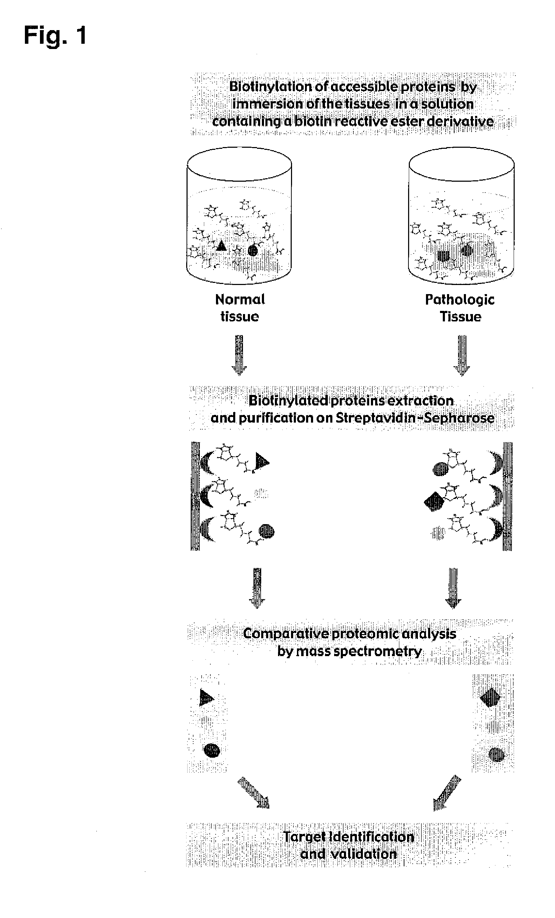

In a preferred embodiment the object is solved by an *in vitro* method for screening specific disease biological markers accessible from the extra cellular space in pathologic tissues comprising the steps of:
- immersion of a normal tissue sample in a solution containing a reactive biotin for labelling proteins; wherein accessible proteins are labelled (biotinylized) by the reactive biotin;
- immersion of a pathologic tissue sample in a solution containing a reactive biotin for labelling proteins; wherein accessible proteins are labelled (biotinylized) by the reactive biotin;
- separate purification of the biotinylized proteins of each of the samples using a streptavidin-bound resin, wherein the streptavidin moiety binds to the biotin group;
- analysis of the labelled proteins or fragments thereof of normal tissue and pathologic tissue, respectively, using liquid chromatography followed by mass spectrometry;
- determination of the differential expression pattern of the labelled proteins in the pathologic tissue samples compared to the normal tissue samples;
- judging that the biotinylized protein(s) having expression in the pathologic tissue sample but which is/are not or essentially not expressed in the corresponding normal tissue is/are accessible biological marker(s) for the given pathologic tissue. Figure 1 summarizes these steps of the method.

**Examples**

**1. Assessment of the immersion time of the tissue in the biotin solution**

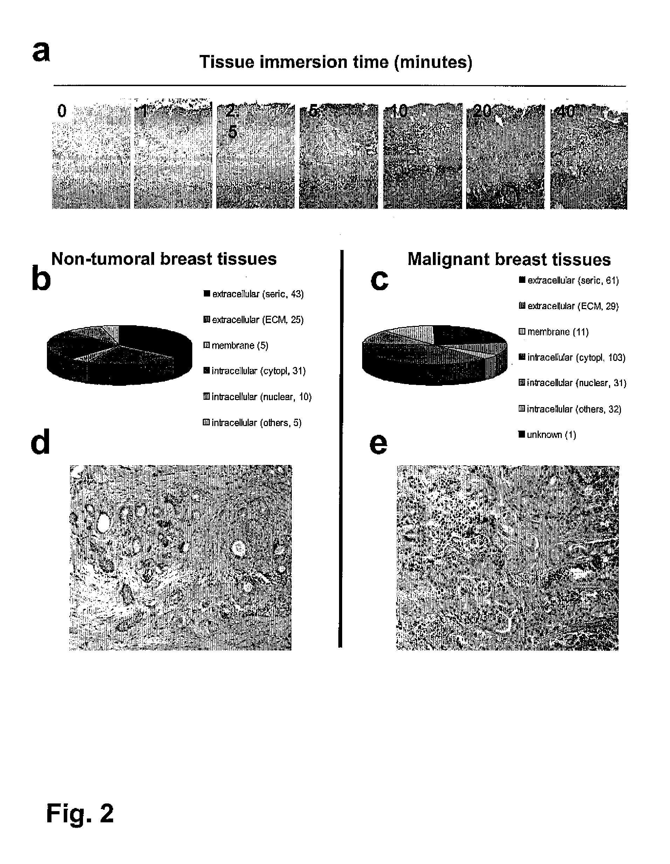

First, the effect of immersion time in the biotin solution on labelling extent and intensity was investigated by histochemistry. Diffusion extent was dependent not only on soaking time, but also on the thickness and composition of the tissue. Increased soaking times (up to 40 minutes) were tested onto breast tissue specimens of variable thicknesses. It was observed that labelling extent, indicative of biotin penetration in the tissues, increased over time (Fig. *2a*). Since a 20-minute immersion time was found appropriate for 2mm-thick human breast tissue slices, this procedure was used in all further experiments.

**2. Expression profiles of accessible proteins in breast carcinomas**

Expression profiles of accessible proteins in 3 ductal and 2 lobular breast carcinomas (clinicopathologic data of the tumors provided in Table 2) and their matched non-tumoral counterpart were determined by mass spectrometry. A gel-free shotgun protein profiling procedure was applied, based on high resolution separation of tryptic peptide digests by nanoscale multidimensional liquid chromatography coupled to electrospray ionization. Micro-sequencing by tandem mass spectrometry allowed analysis and comparison of complex protein mixtures. The number of proteins identified in the biotinylated breast samples (ranging from 127 to 268 in the non-tumoral and cancerous samples, respectively; see pie charts of Fig. 2) was definitely higher than the one found in non-biotinylated, control samples (no more than 10 proteins in normal or tumor samples, data not shown). Proteins found in non-biotinylated samples were most likely "sticky" proteins thatunspecifically bound to the streptavidin beadsand consisted mainly of serum proteins (e.g. human serum albumin, hemoglobin chains and immunoglobulins) and keratins. The distribution of biotin-labelled proteins, identified with the use of stringent analysis parameters, is shown in Fig. *2b* and *2c.* Biotinylated proteins included extra cellular, plasma membrane, and intracellular proteins. The proportion of extra cellular proteins was more important in non tumoral (~57.1%) than in cancerous (~33.5%) samples. This latter result can be largely explained by the enrichment in epithelial cells in the breast cancer tissues (Fig. *2d versus 2e*). As already described in the prior art, i) intracellular proteins are most likely released by damaged cells prior to biotinylation when tissues are sliced, ii) cytoplasmic proteins could be co-purified because of a strong interaction with membrane proteins, and iii) although negatively charged, the long chain biotin esters can enter into the cells (Scheurer, S. B. et al., Identification and relative quantification of membrane proteins by surface biotinylation and two-dimensional peptide mapping. Proteomics 5, 2718-28 (2005); Peirce, M. J. et al., Two-stage affinity purification for inducibly phosphorylated membrane proteins. Proteomics 5, 2417-21 (2005)). Table 1 shows a selected list of accessible proteins expressed either only in tumor tissues, or only in their normal counterparts, or in both. The complete list is available in Table 3.

The comparative analysis of non-tumoral and cancerous breast tissues identified several proteins known to be preferentially localized in the extra cellular compartment. Stromal proteins that are selectively expressed in cancer tissues are prime candidates for tumor targeting strategies because (i) they are expected to be more accessible than intracellular proteins, (ii) they are often present in high quantities, and (iii) tumor cells frequently induce changes in the stromal compartment. This "reactive stroma" creates a permissive and supportive environment contributing to cancer progression (Walker, R. A. The complexities of breast cancer desmoplasia. Breast Cancer Res 3, 143-145 (2001); De Wever, O. & Mareel, M. Role of tissue stroma in cancer cell invasion. J Pathol 200, 429-447 (2003)). For these reasons, identification of new stromal proteins specifically involved in cancer is of high interest. Searching for accessible extra cellular matrix (ECM) proteins, versican and fibronectin have been identified as being systematically expressed only in the breast cancer microenvironment (Table 1). Among other potential extra cellular biological markers, periostin was more frequently detected in the cancerous tissues analyzed. Further, the *in situ* expression of some of these potential markers was investigated by immunohistochemistry in a series of human breast cancers together with their normal counterparts.

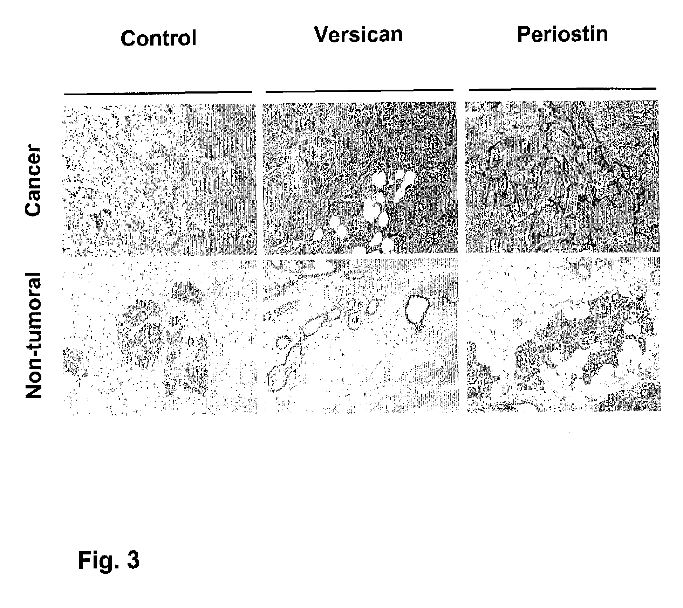

Versican, a large proteoglycan secreted by stromal cells in the ECM, is a recognized cell adhesion and motility modulator that may facilitate tumor cell invasion and metastasis (Yamagata, M., et al., Regulation of cell-substrate adhesion by proteoglycans immobilized on extra cellular substrates. J Biol Chem 264, 8012-8 (1989); Evanko, S. P. et al., Formation of hyaluronan- and versican-rich pericellular matrix is required for proliferation and migration of vascular smooth muscle cells. Arterioscler Thromb Vasc Biol 19, 1004-13 (1999); Ricciardelli, C. et al., Regulation of stromal versican expression by breast cancer cells and importance to relapse-free survival in patients with node-negative primary breast cancer. Clin Cancer Res 8, 1054-60 (2002)). Anti-versican immunoreactivity is increased in the peritumoral stromal matrices of breast (Suwiwat, S. et al., Expression of extra cellular matrix components versican, chondroitin sulfate, tenascin, and hyaluronan, and their association with disease outcome in node-negative breast cancer. Clin Cancer Res 10, 2491-8 (2004)), lung (Pirinen, R. et al., Versican in nonsmall cell lung cancer: relation to hyaluronan, clinicopathologic factors, and prognosis. Hum Pathol 36, 44-50 (2005)), prostate (Ricciardelli, C. et al., Elevated levels of versican but not decorin predict disease progression in early-stage prostate cancer. Clin Cancer Res 4, 963-71 (1998)), and colon (Mukaratirwa, S. et al., Versican and hyaluronan expression in canine colonic adenomas and carcinomas: relation to malignancy and depth of tumour invasion. J Comp Pathol 131, 259-70 (2004)) cancers. In addition, increased accumulation of versican in the stroma surrounding breast cancer cells is associated with a higher risk of relapse in node-negative, primary breast cancer (Ricciardelli, C. et al. Regulation of stromal versican expression by breast cancer cells and importance to relapse-free survival in patients with node-negative primary breast cancer. Clin Cancer Res 8, 1054-60 (2002); Suwiwat, S. et al., Expression of extra cellular matrix components versican, chondroitin sulfate, tenascin, and hyaluronan, and their association with disease outcome in node-negative breast cancer. Clin Cancer Res 10, 2491-8 (2004)). Fig.3 shows representative examples of anti-versican immunostaining in human breast tissues. While versican expression around normal breast glands and ducts was generally absent, the protein harbored strong immunoreactivity in the stromal compartment of the 5 different breast cancer lesions analyzed. These results as well as those from the above-referenced literature are thus fully consistent with the mass spectrometry analysis of the present studies detecting versican only in neoplastic tissues. In view of all these data, versican is proposed as a potential target protein for the treatment of human breast cancer.

Periostin is a soluble, secreted ECM-associated protein (Takeshita, S. et al., Osteoblast-specific factor 2: cloning of a putative bone adhesion protein with homology with the insect protein fasciclin I. Biochem J 294 ( Pt 1), 271-8 (1993)) that localizes with integrins at sites of focal adhesion, thus suggesting a contribution of this protein to cell adhesion and motility (Gillan, L. et al. Periostin secreted by epithelial ovarian carcinoma is a ligand for alpha(V)beta(3) and alpha(V)beta(5) integrins and promotes cell motility. Cancer Res 62, 5358-64 (2002)). It has been recently shown that human periostin is overexpressed in several cancer types, including colorectal carcinoma (Bao, S. et al., Periostin potently promotes metastatic growth of colon cancer by augmenting cell survival via the Akt/PKB pathway. Cancer Cell 5, 329-39 (2004); Tai, I. T. et al., Periostin induction in tumor cell line explants and inhibition of in vitro cell growth by anti-periostin antibodies. Carcinogenesis 26, 908-15 (2005)), breast adenocarcinoma (Shao, R. et al., Acquired expression of periostin by human breast cancers promotes tumor angiogenesis through up-regulation of vascular endothelial growth factor receptor 2 expression. Mol Cell Biol 24, 3992-4003 (2004)), non-small cell lung carcinoma (Sasaki, H. et al., Expression of Periostin, homologous with an insect cell adhesion molecule, as a prognostic marker in non-small cell lung cancers. Jpn J Cancer Res 92, 869-73 (2001)), glioblastoma (Lal, A. et al., A public database for gene expression in human cancers. Cancer Res 59, 5403-7 (1999)), and epithelial ovarian carcinoma (Ismail, R. S. et al., Differential gene expression between normal and tumor-derived ovarian epithelial cells. Cancer Res 60, 6744-9 (2000)). In addition, periostin overexpression in cancer lesions may be associated with advanced disease (Bao, S. et al., Periostin potently promotes metastatic growth of colon cancer by augmenting cell survival via the Akt/PKB pathway. Cancer Cell 5, 329-39 (2004)) and poor outcome (Sasaki, H. et al., Expression of Periostin, homologous with an insect cell adhesion molecule, as a prognostic marker in non-small cell lung cancers. Jpn J Cancer Res 92, 869-73 (2001)). Periostin may facilitate tumor invasion and metastasis by promoting angiogenesis (Bao, S. et al., Periostin potently promotes metastatic growth of colon cancer by augmenting cell survival via the Akt/PKB pathway. Cancer Cell 5, 329-39 (2004); Shao, R. et al., Acquired expression of periostin by human breast cancers promotes tumor angiogenesis through up-regulation of vascular endothelial growth factor receptor 2 expression. Mol Cell Biol 24, 3992-4003 (2004)). Given the suspected role of periostin in tumor metastasis, this secreted protein represents an attractive cancer biological marker and target. In the current study, protein expression profiles obtained in the 5 breast cancer and matched non-tumoral tissues analyzed revealed that periostin was among the most frequently extra cellular proteins identified in the tumor samples (5 out of the 5 breast cancers evaluated). This was further confirmed by immunohistochemical experiments demonstrating an up-regulation of periostin in the stroma associated with all 5 breast cancers analyzed in this study, as compared with the stroma associated with the non-tumoral breast glandular/ductal compartment.

**3. Figure legends**

Fig. 1. Schematic representation of a method for the identification of accessible proteins in human pathologic tissues. *Ex vivo* biotinylation of normal and diseased human tissue samples is achieved by immersion of the tissues into a solution containing a reactive ester derivative of biotin that labels free primary amines. Biotinylated proteins are extracted with strong detergents, captured on streptavidin, and submitted to on-resin proteolytic digestion. Labelled proteins are finally sequenced by tandem mass spectrometry. Differentially identified proteins are validated for example by immunohistochemical analysis.

Fig. 2: *a* : Evaluation of increasing immersion times on the depth of tissue biotinylation. Thin slices of breast cancer tissues were soaked in the biotinylation solution for various periods of time, as indicated. Extent of tissue biotinylation was assessed using histochemistry. Histochemical experiments were carried out with the use of the ABC Vectastain kit, according to the manufacturer's directions (Vector Laboratories, Burlingame, CA, USA). Original magnification: x100. *b-c* : Pie charts representing the proportions of extra cellular and membrane proteins (blue portions) *versus* intracellular proteins (red portions) in biotinylated non-tumoral (*b*) and cancerous (*c*) breast tissues. The number of identified proteins is represented in brackets. Only the principal, known localization of each protein identified was taken into account to draw the pie charts. *d-e* : Representative examples of tissue sections from biotinylated non-tumoral (*d*) and cancerous (*e*) breast tissue samples histochemically stained with streptavidin-peroxidase conjugates. The histochemical procedure was performed as described above. Original magnification: x100 Fig. 2: *a-b*: Paraffin sections of biotinylated non tumoral (*a*) and cancerous (*b*) breast tissue samples revealed histochemically with avidin HRP using the ABC Vectastain kit, according to the manufacturer's directions (Vector Laboratories, Burlingame, CA). *c-d*: Pie charts representing the proportions of extra cellular and membrane proteins (blue portions) *versus* intracellular proteins (red portions) in biotinylated non tumoral (*c*) and cancerous (*d*) breast tissues. The number of identified proteins is represented under brackets. Only the principal, known localization of each protein identified was taken into account to draw the pie charts.

Fig. 3: Representative examples of versican and periostin expression, as assessed by immunohistochemistry, in cancerous and matched non-tumoral human breast cancer tissues. Paraffin sections of human breast tissues were subjected to immunoperoxidase. Original magnification: x100.

**4. Tissue harvesting**

Fresh breast tissue samples were obtained from mastectomy specimens. Tissue samples were immmediately cut in small slices and then soaked into the biotinylation solution (1 mg.ml⁻¹ of EZ-link Sulfo NHS-LC biotin (Pierce) dissolved extemporaneously in PBS [pH 7.4]). Each biotinylation reaction was stopped by a 5 minute incubation in a primary amine-containing buffer solution (e.g. 50mM Tris [pH 7.4]). Tissue samples were then snap-frozen in liquid nitrogen, except for a tiny portion of each sample that was directly immersed in formalin and then processed for further histological and histochemical investigations. Controls included adjacent tissue slices immersed in PBS.

**5. Histochemistry and immunohistochemistry**

In order to assess the diffusion depth of reactive biotin ester derivatives in the biotinylated tissues, formalin-fixed, paraffin-embedded breast tissue sections were incubated with avidin-peroxidase conjugates with the use of the Vectastain ABC kit (Vector Labortories, Burlingame, CA, USA), according to the manufacturer's instructions. Immunohistochemical experiments were performed as previously described by Waltregny et al. (Waltregny, D. et al. Prognostic value of bone sialoprotein expression in clinically localized human prostate cancer. J Natl Cancer Inst 90, 1000-1008 (1998)). For the immunohistochemical detection of versican, antigen retrieval was performed by incubating the slides with chondroitinase. Anti-versican (clone 12C5, Developmental Studies Hybridoma Bank at the University of Iowa, Iowa City, IA, USA) and anti-periostin (rabbit polyclonal, BioVendor GmbH, Heidelberg, Germany) antibodies were applied onto the sections at a dilution of 1:200 and 1:1000, respectively. Control experiments included omission of the primary antibody in the procedure.

**6. Sample processing**

Pulverization of frozen biotinylated biopsies was performed using a Mikro-Dismembrator U (Braun Biotech, Melsungen, Germany) and generated tissue powder that was resuspended in a buffer containing 1 % NP40, 0.5% DOC, 0.1% SDS and a protease inhibitor cocktail (Complete, Roche Diagnostics, Mannheim, Germany). Lysates were sonicated (2x30") and subjected to a preclearing step by incubation with protein G-sepharose beads (Amersham Biosciences). This step was designed to limit immunoglobulins detection by mass spectrometry, but did not hinder detection of low abundant proteins (data not shown). Lysates were then boiled for 5 minutes and samples were filtered onto 0.45µm acrodiscs to remove remaining sepharose beads and fatty aggregates. Protein concentration was determined using the BCA protein assay reagent kit (Pierce Chemical Co.). Equal amounts of proteins were mixed with streptavidin-sepharose slurry (Amersham Biosciences), and equilibrated by three washes in buffer A (1% NP40 and 0.1% SDS in PBS). Fifty µl of beads were used per mg of biotinylated proteins, and protein binding was allowed for 1 hour at room temperature in a rotating mixer. The resin was then washed twice with buffer A, twice with buffer B (0.1% NP40, 1M NaCl in PBS) and once with ammonium bicarbonate 50mM (pH 7.8). Binding of the biotinylated proteins onto the resin and washing efficiency was checked by SDS-PAGE, and further either by Coomassie blue staining or by blotting for subsequent detection of biotin by streptavidin-horseradish peroxidase (data not shown). On-resin digestion was carried out overnight at 37°C with modified porcine trypsin (Promega) in 100µl final volume of NH₄HCO₃ (pH 7.8).

**7. Mass spectrometry**

Supernatant was collected, protein concentration was determined and once evaporated, the digested products were redissolved in 0.1 % formic acid. Peptide separation by reverse-phase liquid chromatography was performed on an "Ultimate LC" system (LC Packings) completed with a Famos autosampler and a Swichos II Microcolumn switching device for sample clean-up, fractionation and preconcentration. Sample (20µL at 0.25µg/µL 0.1% formic acid) was first trapped on a SCX micro pre-column (500µM internal diameter, 15mm length, packed with MCA50 bioX-SCX 5µm; LC Packings) at a flow rate of 200nL/min followed by a micro pre-column cartridge (300µM i.d., 5mm length, packed with 5µm C18 PepMap100 ; LC Packings). After 5 min the precolumn was connected with the separating nanocolumn (75µm i.d., 15cm length, packed with 3µm C18 PepMAp100 ; LC Packings) equilibrated in mobile phase A (0,1% formic acid in 2:98 of acetonitrile:degassed milliQ water). A linear elution gradient was applied with mobile phase B (0.1% formic acid in 80:20 of acetonitrile:degassed milliQ water) from 10% to 40% spanning on 95 minutes. The outlet of the LC system was directly connected to the nano electrospray source of an Esquire HCT ion trap mass spectrometer (Bruker Daltonics, Germany). Mass data acquisition was performed in the mass range of 50-2000 m/z using the standard-enhanced mode (8100 m/z per second). For each mass scan, a data dependant scheme picked the 4 most intense doubly or triply charged ions to be selectively isolated and fragmented in the trap. The resulting fragments were analyzed using the Ultra Scan mode (m/z range of 50-3000 at 26000m/z per second). SCX-trapped peptides were stepwise eluted with 4 salt concentrations (20mM, 60mM, 200mM and 800mM), each followed by the same gradient of mobile phase B.

**8. Database Searching**

The minimally redundant database of human protein sequences Swiss Prot (release 47.8, SIB; Switzerland) was chosen (Nesvizhskii, A. I. & Aebersold, R. Interpretation of Shotgun Proteomic Data: The Protein Inference Problem. Mol Cell Proteomics 4, 1419-1440 (2005)). Raw data were analysed and formatted (Data Analysis software; Bruker) for subsequent protein identification using the Swiss Prot human protein database through the MS/MS ion search algorithm of the Mascot search engine (Perkins, D. N. et al., Electrophoresis 20, 3551-67 (1999)), running on a multi-processor computer cluster. The mass tolerance of precursor and fragmented ions were set at 0.6 and 0.3 respectively; allowed modifications were partial oxidization of methionines. Up to 2 misscuts were allowed. The absolute probability (P) was set to 0.01, i.e. less than 1% probability of a random match. The MudPIT scoring was used, while discriminating peptides with ion scores inferior to 15 and requesting protein hits to have at least one newly sequenced peptide ("bold red" request feature).

**Table 1: Selection of accessible proteins.**

| Protein Name | SwissProt Accession No | Non-tumoral breast | Cancerous breast |
|---|---|---|---|
| Serum amyloid A-4 protein | P35542 | 2 | 0 |
| Collagen type I alpha 2 | P08123 | 3 | 1 |
| Keratin, type I cytoskeletal 9 | P35527 | 4 | 2 |
| Keratin, type II cytoskeletal 1 | P04264 | 5 | 3 |
| Vimentin | P08670 | 5 | 5 |
| Bone/cartilage proteoglycan | P21810 | 5 | 5 |
| Lumican precursor | P51884 | 5 | 5 |
| Hemoglobin beta chain | P68871 | 5 | 5 |
| Hemopexin | P02790 | 5 | 5 |
| Decorin | P07585 | 5 | 5 |
| Collagen type VI alpha 3 | P12111 | 5 | 5 |
| Osteoglycin | P20774 | 5 | 4 |
| Periostin precursor | Q15063 | 1 | 5 |
| Keratin, type I cytoskeletal 19 | P08727 | 0 | 4 |
| Endoplasmin precursor 94kDa glucose-regulated protein | P14625 | 0 | 4 |
| Versican core protein precursor | P13611 | 0 | 4 |
| Fibronectin precursor | P02751 | 0 | 4 |
| Lamin B1 | P20700 | 0 | 3 |
| Anterior gradient protein 2 homolog precursor | 095994 | 0 | 3 |
| Keratin, type I cytoskeletal 18 | P05783 | 0 | 3 |

Some proteins were detected mainly either in non tumoral, or in cancerous, or both in non tumoral and in cancerous tissues. The values indicate in how many of the 5 *ex vivo* biotinylated breast cancer lesions and matched non-tumoral tisses analyzed, each of the proteins listed was detected.

**Table 2: Clinicopathological status of the breast cancer lesions analyzed in the study.**

| # | Age | Tumor size (cm) | Type | Grade | Nodal status | ER | PR | ErbB2 |
|---|---|---|---|---|---|---|---|---|
| 1 | 74 | 2.5 | Infiltrating ductal | 2 | N0 | + | + | - |
| 2 | 56 | 4 | Infiltrating lobular | 2 | N0 | + | + | - |
| 3 | 56 | 1.8 | Infiltrating ductal | 3 | N+ | + | + | - |
| 4 | 74 | 1.5 | Infiltrating lobular | 2 | N0 | + | - | - |
| 5 | 77 | 2.2 | Infiltrating ductal | 3 | N0 | - | - | + |

Cancerous and matched non tumoral tissues from each mastectomy specimen were soaked in PBS or EZ-link Sulfo-NHS-LC-biotin, and then flash frozen in liquid nitrogen for subsequent analyses. (ER: Estrogen-receptor status. PR: progesterone receptor status. The grading system used is based on the Bloom classification modified by Elston.)

**Table 3: Complete list of proteins identified by nanoLC-ESI-MS/MS analysis with stringent parameters. The numbers indicate the number of patients for which proteins were found either in cancerous or in non-tumoral tissues.**

| Protein Name | SwissProt ID | Non tumoral | Cancer |
|---|---|---|---|
| Alpha-1-antitrypsin precursor | P01009 | 5 | 5 |
| Ig alpha-1 chain | P01876 | 5 | 5 |
| Apolipoprotein A I | P02647 | 5 | 5 |
| Serum albumin precursor | P02768 | 5 | 5 |
| Serotransferrin precursor | P02787 | 5 | 5 |
| Vimentin | P08670 | 5 | 5 |
| Bone/cartilage proteoglycan | P21810 | 5 | 5 |
| Lumican precursor | P51884 | 5 | 5 |
| Hemoglobin beta chain | P68871 | 5 | 5 |
| Hemopexin | P02790 | 5 | 5 |
| Decorin | P07585 | 5 | 5 |
| Collagen type VI alpha 3 | P12111 | 5 | 5 |
| Immunoglobulin kappa light chain | P01834 | 5 | 5 |
| Hemoglobin alpha chain | P69905 | 5 | 5 |
| Osteoglycin | P20774 | 5 | 4 |
| Keratin, type II cytoskeletal | P04264 | 5 | 3 |
| Collagen alpha 1 XIV chain | Q05707 | 4 | 5 |
| Prolargin precursor | P51888 | 4 | 5 |
| Macroglobulin alpha 2 | P01023 | 4 | 5 |
| Fibrinogen beta chain | P02675 | 4 | 5 |
| Ig lambda chain C regions | P01842 | 4 | 5 |
| Fibrinogen gamma chain precursor | P02679 | 4 | 5 |
| Actin cytoplasmic 1 Beta | P60709 | 4 | 5 |
| Complement C3 precursor | P01024 | 4 | 4 |
| Collagen alpha1 VI chain precursor | P12109 | 4 | 4 |
| Clusterin precursor | P10909 | 4 | 4 |
| Keratin, type I cytoskeletal 10 | P13645 | 4 | 3 |
| Transthyretin precursor | P02766 | 4 | 3 |
| Alpha 1 antichymotrypsin | P01011 | 4 | 3 |
| Keratin, type I cytoskeletal 9 | P35527 | 4 | 2 |
| Ig mu chain C region | P01871 | 4 | 2 |
| Annexin A2 | P07355 | 3 | 5 |
| lg kappa chain V-III region SIE | P01620 | 3 | 4 |
| lg kappa chain V-IV region Len | P01625 | 3 | 4 |
| Lamin A/C | P02545 | 3 | 4 |
| Ig heavy chain V-III region BRO | P01766 | 3 | 3 |
| Lactotransferrin precursor | P02788 | 3 | 3 |
| Keratin type II cytoskeletal 3 | P12035 | 3 | 3 |
| Hemoglobin delta chain | P02042 | 3 | 3 |
| Actin, aortic smooth muscle Alpha-actin-2 | P62736 | 3 | 2 |
| Immunoglobin Gm 1 | P01857 | 3 | 2 |
| Keratin 2E | P35908 | 3 | 2 |
| Serum amyloid P-component precursor | P02743 | 3 | 2 |
| Histone H1t | P22492 | 3 | 1 |
| Collagen type I alpha 2 | P08123 | 3 | 1 |
| Apolipoprotein D precursor | P05090 | 3 | 1 |
| Immunoglobulin J chain | P01591 | 3 | 1 |
| Haptoglobin-related protein precursor | P00739 | 3 | 1 |
| Histone H4 | P62805 | 2 | 5 |
| Immunoglobulin Am2 | P01877 | 2 | 5 |
| Haptoglobin precursor | P00738 | 2 | 4 |
| Keratin, type II cytoskeletal 8 | P05787 | 2 | 4 |
| Gelsolin precursor | P06396 | 2 | 4 |
| Tubulin alpha | P68363 | 2 | 4 |
| Collagen alpha 2(VI) chain precursor | P12110 | 2 | 4 |
| Tropomyosin 1 alpha chain | P09493 | 2 | 3 |
| Antithrombin III | P01008 | 2 | 3 |
| Triosephosphate isomerase 1 | P60174 | 2 | 3 |
| Ig kappa chain V-II region Cum | P01614 | 2 | 2 |
| Ig lambda chain V-III region LOI | P80748 | 2 | 2 |
| lg kappa chain V-I region CAR | P01596 | 2 | 2 |
| Asporin precursor | Q9BXN1 | 2 | 2 |
| Histone H2B a/g/h/k/l | P62807 | 2 | 2 |
| Prolactin-inducible protein precursor | P12273 | 2 | 2 |
| Alpha-1 acid glycoprotein | P02763 | 2 | 2 |
| Vitamin D binding protein | P02774 | 2 | 2 |
| Ig kappa chain V-I region AG | P01593 | 2 | 2 |
| Fibrinogen alpha chain precursor | P02671 | 2 | 2 |
| Vitronectin | P04004 | 2 | 2 |
| L-lactate dehydrogenase A chain | P00338 | 2 | 2 |
| Dermatopontin precursor | Q07507 | 2 | 1 |
| Alpha-2-HS-glycoprotein precursor, Fetuin | P02765 | 2 | 1 |
| Ceruloplasmin precursor | P00450 | 2 | 1 |
| Plasma protease C1 inhibitor precursor | P05155 | 2 | 1 |
| Apolipoprotein E | P02649 | 2 | 1 |
| Apolipoprotein C-III precursor | P02656 | 2 | 1 |
| Ig heavy chain V-III region HIL | P01771 | 2 | 1 |
| Tetranectin precursor | P05452 | 2 | 1 |
| Serum amyloid A-4 protein (candidate of metastasis 1) | P35542 | 2 | 0 |
| Periostin precursor | Q15063 | 1 | 5 |
| Tubulin beta-2 chain | P07437 | 1 | 4 |
| Tropomyosin beta chain | P07951 | 1 | 4 |
| Filamin A | P21333 | 1 | 4 |
| Keratin, type II cytoskeletal 7 | P08729 | 1 | 4 |
| Myosin heavy polypeptide 9 non-muscle | P35579 | 1 | 4 |
| Peptidyl-prolyl cis-trans isomerase A | P62937 | 1 | 4 |
| 78 kDa glucose-regulated protein precursor | P11021 | 1 | 4 |
| 14-3-3 protein zeta/delta | P63104 | 1 | 4 |
| Pyruvate kinase, isozymes M1/M2 | P14618 | 1 | 3 |
| Synaptic vesicle membrane protein VAT-1 homolog | Q99536 | 1 | 3 |
| Annexin A5 | P08758 | 1 | 3 |
| Complement factor H precursor | P08603 | 1 | 3 |
| Histone 1 H2AE | P28001 | 1 | 3 |
| Annexin A1 | P04083 | 1 | 3 |
| Fibulin-1 precursor | P23142 | 1 | 3 |
| Alpha enolase | P06733 | 1 | 3 |
| Heat shock 70kDa | P08107 | 1 | 2 |
| Vinculin Metavinculin | P18206 | 1 | 2 |
| Myosin-11 | P35749 | 1 | 2 |
| Actin, alpha cardiac | P68032 | 1 | 2 |
| RNA Polymerase I and transcript release factor PTRF | Q6NZ12 | 1 | 2 |
| Ig gamma2 chain C | P01859 | 1 | 2 |
| Hemoglobin gamma-1 chain | P69891 | 1 | 2 |
| Apolipoprotein A IV | P06727 | 1 | 2 |
| Histidine rich glycoprotein | P04196 | 1 | 2 |
| lg kappa chain V-I region DEE | P01597 | 1 | 2 |
| Glutathione S-transferase P | P09211 | 1 | 2 |
| ATP synthase beta chain, mitochondrial precursor | P06576 | 1 | 2 |
| Profilin-1 | P07737 | 1 | 2 |
| 14-3-3 protein gamma | P61981 | 1 | 2 |
| Prolactin receptor associated protein | P06703 | 1 | 2 |
| Thioredoxin | P10599 | 1 | 2 |
| AMBP protein precursor | P02760 | 1 | 2 |
| Mast cell carboxypeptidase A precursor | P15088 | 1 | 1 |
| Disheveled associated activator of morphogenesis 2 | Q86T65 | 1 | 1 |
| Serum amyloid A protein precursor | P02735 | 1 | 1 |
| Keratin, type II cytoskeletal 6A | P02538 | 1 | 1 |
| Myosin light chain 1, slow-twitch muscle A isoform | P14649 | 1 | 1 |
| Peroxiredoxin 2 | P32119 | 1 | 1 |
| Ig heavy chain V-III region TIL | P01765 | 1 | 1 |
| Complement factor B precursor | P00751 | 1 | 1 |
| lg kappa chain V-I region Lay | P01605 | 1 | 0 |
| Target of Nesh-SH3 precursor | Q7Z7G0 | 1 | 0 |
| Tubulointerstitial nephritis antigen | Q9GZM7 | 1 | 0 |
| lg lambda chain V-IV region Hil | P01717 | 1 | 0 |
| Zinc-alpha-2-glycoprotein precursor | P25311 | 1 | 0 |
| Receptor-interacting serine/threonine-protein kinase 2 | Q13546 | 1 | 0 |
| Plasminogen precursor | P00747 | 1 | 0 |
| Heparin cofactor II precursor | P05546 | 1 | 0 |
| lg kappa chain V-I region OU | P01606 | 1 | 0 |
| Keratin, type I cytoskeletal 17 | Q04695 | 1 | 0 |
| Carbonic anhydrase 1 | P00915 | 1 | 0 |
| Voltage-dependent L-type calcium channel alpha-1 | Q13698 | 1 | 0 |
| Ras-related protein R-Ras (p23) | P10301 | 1 | 0 |
| Heat shock protein 75 kDa, mitochondrial precursor | Q12931 | 1 | 0 |
| DNA-dependent protein kinase catalytic subunit | P78527 | 1 | 0 |
| Kininogen-1 precursor | P01042 | 1 | 0 |
| Corticosteroid-binding globulin precursor | P08185 | 1 | 0 |
| Complement C4 precursor | P01028 | 1 | 0 |
| Inter-alpha-trypsin inhibitor heavy chain H4 precursor | Q14624 | 1 | 0 |
| Myosin light polypeptide 6 | P60660 | 1 | 0 |
| Keratin, type I cytoskeletal 13 | P13646 | 1 | 0 |
| Endoplasmin precursor 94kDa glucose-regulated protein | P14625 | 0 | 4 |
| Ubiquitin | P62988 | 0 | 4 |
| Versican core protein precursor | P13611 | 0 | 4 |
| Fibronectin precursor | P02751 | 0 | 4 |
| Keratin, type I cytoskeletal 19 | P08727 | 0 | 4 |
| Heterogeneous nuclear ribonucleoproteins A2/B1 | P22626 | 0 | 4 |
| Phosphoglycerate kinase 1 | P00558 | 0 | 4 |
| Protein disulfide-isomerase precursor | P07237 | 0 | 4 |
| Macrophage capping protein | P40121 | 0 | 3 |
| Actinin alpha4 | 043707 | 0 | 3 |
| Glyceraldehyde-3-phosphatedehydrogenase | P04406 | 0 | 3 |
| Heat-shock protein beta-1 | P04792 | 0 | 3 |
| Keratin, type I cytoskeletal 18 | P05783 | 0 | 3 |
| Heat shock protein HSP90 - alphaHSP86 | P07900 | 0 | 3 |
| Heat shock cognate 71 kDa protein | P11142 | 0 | 3 |
| Alpha-actinin 1 | P12814 | 0 | 3 |
| Cofilin-1 | P23528 | 0 | 3 |
| Heat shock 70k | P34931 | 0 | 3 |
| Peroxiredoxin 1 | Q06830 | 0 | 3 |
| Angiotensinogen precursor | P01019 | 0 | 3 |
| Fructose-bisphosphate aldolase A | P04075 | 0 | 3 |
| Protein disulfide-isomerase A3 precursor | P30101 | 0 | 3 |
| 60 kDa heat shock protein mitochondrial precursor | P10809 | 0 | 3 |
| Anterior gradient protein 2 homolog precursor | 095994 | 0 | 3 |
| Cathepsin D | P07339 | 0 | 3 |
| Lamin B1 | P20700 | 0 | 3 |
| Collagen alpha 1 (XII) | Q99715 | 0 | 2 |
| Protein disulfide-isomerase A6 precursor | Q15084 | 0 | 2 |
| ADP-ribosylation factor 1 | P84077 | 0 | 2 |
| Chloride intracellular channel protein 1 | 000299 | 0 | 2 |
| Ribosome-binding protein 1 | Q9P2E9 | 0 | 2 |
| Nascent polypeptide-associated complex alpha subunit | Q13765 | 0 | 2 |
| Rho GDP-dissociation inhibitor 2 | P52566 | 0 | 2 |
| LIM and SH3 domain protein 1 | Q14847 | 0 | 2 |
| Histone H1.5 | P16401 | 0 | 2 |
| Calreticulin precursor | P27797 | 0 | 2 |
| Elongation factor 1-delta | P29692 | 0 | 2 |
| Annexin A6 | P08133 | 0 | 2 |
| 14-3-3 protein beta/alpha | P31946 | 0 | 2 |
| 14-3-3 protein epsilon | P62258 | 0 | 2 |
| Elongation factor 1-alpha | P68104 | 0 | 2 |
| Transgelin | Q01995 | 0 | 2 |
| Talin-1 | Q9Y490 | 0 | 2 |
| Collagen type I alpha 1 | P02452 | 0 | 2 |
| Tubulin beta 2 | P68371 | 0 | 2 |
| Calgizzarin S100C | P31949 | 0 | 2 |
| Macrophage migration inhibitory factor | P14174 | 0 | 2 |
| Heat shock protein HSP 90-beta | P08238 | 0 | 2 |
| Neuroblast differentiation associated protein AHNAK | Q09666 | 0 | 2 |
| Transgelin-2 | P37802 | 0 | 2 |
| Heterogeneous nuclear ribonucleoprotein D0 | Q14103 | 0 | 2 |
| Heterogeneous nuclear ribonucleoprotein K | P61978 | 0 | 2 |
| Raf kinase inhibitor protein | P30086 | 0 | 2 |
| Histone H1.2 | P16403 | 0 | 2 |
| Lamin B2 | Q03252 | 0 | 2 |
| Calnexin precursor | P27824 | 0 | 2 |
| T-plastin | P13797 | 0 | 2 |
| Superoxide dismutase mitochondrial precursor | P04179 | 0 | 2 |
| Tubulin alpha-3 chain | Q71 U36 | 0 | 1 |
| Keratin, type I cytoskeletal 16 | P08779 | 0 | 1 |
| Myristoylated alanine-rich C-kinase substrate | P29966 | 0 | 1 |
| HLA class I histocompatibility antigen | P01893 | 0 | 1 |
| Tubulin beta-6 chain | Q9BUF5 | 0 | 1 |
| Phosphate carrier protein | Q00325 | 0 | 1 |
| Heterogeneous nuclear ribonucleoprotein L | P14866 | 0 | 1 |
| Moesin | P26038 | 0 | 1 |
| Voltage-dependent anion-selective channel protein 3 | Q9Y277 | 0 | 1 |
| lg heavy chain V-III region GAL | P01781 | 0 | 1 |
| Ig kappa chain V-I region BAN | P04430 | 0 | 1 |
| Heterogeneous nuclear ribonucleoprotein C-like | 060812 | 0 | 1 |
| 14-3-3 protein theta (14-3-3 protein tau) | P27348 | 0 | 1 |
| Voltage-dependent anion-selective channel protein 1 | P21796 | 0 | 1 |
| Apolipoprotein-L1 precursor | 014791 | 0 | 1 |
| Tubulin beta-3 chain | Q13509 | 0 | 1 |
| Translin-associated protein X | Q99598 | 0 | 1 |
| Beta-2-glycoprotein I precursor | P02749 | 0 | 1 |
| Signal recognition particle 14 kDa protein | P37108 | 0 | 1 |
| Calmodulin (CaM) | P62158 | 0 | 1 |
| HLA class II histocompatibility antigen, gamma chain | P04233 | 0 | 1 |
| Splicing factor, arginine/serine-rich 1 | Q07955 | 0 | 1 |
| Ig kappa chain V-I region Wes | P01611 | 0 | 1 |
| Glutathione S-transferase Mu 3 | P21266 | 0 | 1 |
| Heterogenous nuclear ribonucleoprotein U | Q00839 | 0 | 1 |
| Nucleoside diphosphate kinase A | P15531 | 0 | 1 |
| Selenoprotein P precursor | P49908 | 0 | 1 |
| Apolipoprotein B-100 precursor | P04114 | 0 | 1 |
| Lactoylglutathione lyase | Q04760 | 0 | 1 |
| Peptidyl-prolyl cis-trans isomerase B precursor | P23284 | 0 | 1 |
| Heterogeneous nuclear ribonucleoprotein A3 | P51991 | 0 | 1 |
| Actin-like protein 2 | P61160 | 0 | 1 |
| Cysteine and glycine-rich protein 1 | P21291 | 0 | 1 |
| ATP synthase alpha chain | P25705 | 0 | 1 |
| Prohibitin | P35232 | 0 | 1 |
| Spectrin beta chain | Q01082 | 0 | 1 |
| Caldesmon | Q05682 | 0 | 1 |
| Ras-related protein Rab-35 | Q15286 | 0 | 1 |
| Tropomyosin alpha 4 chain | P67936 | 0 | 1 |
| Tropomyosin alpha 3 chain | P06753 | 0 | 1 |
| Protein disulfide-isomerase A4 precursor | P13667 | 0 | 1 |
| Histone H1.4 | P10412 | 0 | 1 |
| Myosin regulatory light chain 2 | P19105 | 0 | 1 |
| Stress-70 protein, mitochondrial precursor | P38646 | 0 | 1 |
| HLA class I histocompatibility antigen | P13747 | 0 | 1 |
| 40S ribosomal protein S28 | P62857 | 0 | 1 |
| Isocitrate dehydrogenase | 075874 | 0 | 1 |
| Tenascin precursor | P24821 | 0 | 1 |
| Thymidine phosphorylase precursor | P19971 | 0 | 1 |
| L-plastin | P13796 | 0 | 1 |
| HLA class II histocompatibility antigen | P01903 | 0 | 1 |
| Far upstream element binding protein 2 | Q92945 | 0 | 1 |
| Isocitrate dehydrogenase | P48735 | 0 | 1 |
| General vesicular transport factor p115 | 060763 | 0 | 1 |
| Spectrin alpha chain, brain | Q13813 | 0 | 1 |
| ERO1-like protein alpha precursor | Q96HE7 | 0 | 1 |
| Transaldolase | P37837 | 0 | 1 |
| Vesicle-associated membrane protein-associated protein A | Q9P0L0 | 0 | 1 |
| Glucosidase II beta subunit precursor | P14314 | 0 | 1 |
| Perilipin | 060240 | 0 | 1 |
| Laminin alpha-4 chain precursor | Q16363 | 0 | 1 |
| Ig kappa chain V-III region B6 | P01619 | 0 | 1 |
| Fatty acid-binding protein | P15090 | 0 | 1 |
| Alpha-1B-glycoprotein precursor | P04217 | 0 | 1 |
| Prothrombin precursor | P00734 | 0 | 1 |
| Complement factor I precursor | P05156 | 0 | 1 |
| Eukaryotic translation initiation factor 1 | P41567 | 0 | 1 |
| Electron transfer flavoprotein alpha-subunit | P13804 | 0 | 1 |
| Calgranulin B | P06702 | 0 | 1 |
| Transcription intermediary factor 1-beta | Q13263 | 0 | 1 |
| Calpastatin | P20810 | 0 | 1 |
| Calgranulin A | P05109 | 0 | 1 |
| Toll-interacting protein | Q9H0E2 | 0 | 1 |
| Galectin-1 | P09382 | 0 | 1 |
| Keratin, type I cytoskeletal 15 | P19012 | 0 | 1 |
| Elongation factor 1-bet | P24534 | 0 | 1 |
| Glutathione S-transferase Mu 1 | P09488 | 0 | 1 |
| Adenylate kinase isoenzyme 2 | P54819 | 0 | 1 |
| Heterogeneous nuclear ribonucleoprotein A1 | P09651 | 0 | 1 |
| Mucin-1 precursor | P15941 | 0 | 1 |
| Peroxiredoxin 5 | P30044 | 0 | 1 |
| Nucleophosmin | P06748 | 0 | 1 |
| Histone H2A.z | P17317 | 0 | 1 |
| 40S ribosomal protein SA | P08865 | 0 | 1 |
| Actin-related protein 2/3 complex subunit 3 | 015145 | 0 | 1 |
| Nucleolin | P19338 | 0 | 1 |
| Transitional endoplasmic reticulum ATPase | P55072 | 0 | 1 |
| Aconitate hydratase mitochondrial precursor | Q99798 | 0 | 1 |
| Poly(rC)-binding protein 1 | Q15365 | 0 | 1 |
| F-actin capping protein alpha-1 subunit | P52907 | 0 | 1 |

## Claims

1. An in-vitro method for screening specific disease biological markers accessible from the extra cellular space in pathologic tissues comprising the steps of:
- immersion of a pathologic tissue sample in a solution containing a labelling reagent for labelling proteins, wherein accessible proteins are labelled by the labelling reagent;
- purification of the labelled proteins;
- analysis of the labelled proteins or fragments thereof;
- determination of the differential expression pattern of the labelled proteins in pathological tissue samples compared to corresponding and/or unrelated normal tissues;
- judging that the labelled protein(s) having higher expression in the pathologic tissue sample compared to corresponding and/or unrelated normal tissue samples or being expressed more frequently in respective pathologic tissue samples compared to corresponding and/or unrelated normal tissue samples is/are accessible biological marker(s) for pathologic tissue.

2. The method according to claim 1, comprising the steps of
- immersion of a normal tissue sample in a solution containing a labelling reagent for labelling proteins; wherein accessible proteins are labelled by the labelling reagent;
- immersion of a pathologic tissue sample in a solution containing a labelling reagent for labelling proteins, wherein accessible proteins are labelled by the labelling reagent;
- separate purification of the labelled proteins of each of the samples;
- analysis of the labelled proteins or fragments thereof of normal tissue and pathologic tissue, respectively;
- determination of the differential expression pattern of the labelled proteins in the pathological tissue samples compared to the normal tissue samples;
- judging that the labelled protein(s) having higher expression in the pathologic tissue sample compared to the normal tissue sample or being expressed more frequently in respective pathologic tissue samples compared to the normal tissue sample is/are accessible biological marker(s) for pathologic tissue.

3. The method according to claim 1 or 2, wherein it is judged that the labelled protein(s) having expression in the pathologic tissue sample but which is/are not or essentially not expressed in the normal tissue is/are accessible biological marker(s) for pathologic tissue.

4. The method according to any one of claims 1 to 3, wherein the labelling reagent for labelling proteins is a reactive biotin, preferably a biotin reactive ester derivative.

5. The method according to any one of claims 1 to 4, wherein the purification step makes use of the label of the labelled proteins as selective marker.

6. The method according to any one of claims 1 to 5, wherein the label is a biotin residue and wherein purification is performed using streptavidin bound to a resin, wherein the biotin-labelled proteins are bound to the resin via streptavidin.

7. The method according to any one of claims 1 to 6, wherein after the purification step the labelled proteins are cleaved to peptides, preferably by proteolytic digestion.

8. The method according to any one of claims 1 to 7, wherein the analysis step comprises mass spectrometry, preferably microsequencing by tandem mass spectrometry.

9. The method according to any one of claims 1 to 8, wherein the pathologic tissue sample is derived from tissues selected from the group consisting of tumor tissue, inflamed tissue, atheromatotic tissue or resulting from degenerative, metabolic and genetic diseases.

10. The method according to any one of claims 1 to 9, wherein accessibility of the biological markers refers to being accessible for substances from the extra cellular space.

11. The method according to claim 10, wherein the substances are selected from the group consisting of antibodies, antibody fragments, drugs, prodrugs, ligands, biotin, and derivatives and conjugates thereof, preferably conjugates of antibodies or antibody fragments with drugs or prodrugs.

12. The method according to any one of claims 1 to 11, wherein biological markers are proteins or polypeptides, which are expressed in the given pathologic tissue and not expressed in the normal tissue, or are expressed on a higher level in the given pathologic tissue than in the normal tissue, wherein the biological marker indicates a pathologic condition compared to the normal physiologic condition of the corresponding normal tissue.

13. Use of the method according to any one of claims 1 to 12 for the manufacture of a medicament for therapeutic and/or preventive treatment of a human or animal disease, wherein a high-affinity ligand directed against a biological marker for pathological tissue is comprised in the medicament, wherein said biological marker is accessible for high-affinity ligands from the extra cellular space.

14. Use of the method according to any one of claims 1 to 12 for the development of techniques for the detection of pathologic tissues.

15. Use of the method according to any one of claims 1 to 12 for the development of a medicament based on the use of specific ligands, preferably antibodies and antibody-drug conjugates, for therapeutic and/or preventive treatment of human or animal diseases.

16. Use of the method according to any one of claims 1 to 12 for the screening of accessible biological markers of a pathologic tissue of an individual patient for the development of an individual treatment protocol.

17. A method for therapeutic and/or preventive treatment of a human or animal disease, wherein a high-affinity ligand directed against a biological marker for pathological tissue is used, wherein said biological marker is accessible for high-affinity ligands from the extra cellular space, wherein the method is comprising the procedure according to any one of claims 1 to 12.
